# EUROPEAN PATENT APPLICATION

(11) **EP 4 509 148 A1**
(43) Date of publication of application: **19.02.2025**
(21) Application number: 23191394.8
(22) Date of filing: 14.08.2023
(51) Int. Cl.: A61M 1/06, A61J 13/00, A61M 1/00

(54) **BREAST MILK COLLECTOR AND METHOD**

(71) Applicant: Koninklijke Philips N.V., 5656 AG Eindhoven (NL)
(72) Inventor: BARINK, Marco, Eindhoven (NL); VAN DER KOOI, Johannes Tseard, Eindhoven (NL); VAN DEN BIJGAART, Adrianus Wilhelmus Dionisius Maria, Eindhoven (NL); VAN BENTUM, Jesper William, Eindhoven (NL)
(74) Representative: Philips Intellectual Property & Standards

(57) **Abstract**

Provided is a passive breast milk collector (10) for collecting milk from a mother's breast. The breast milk collector comprises a container (11). The breast milk collector is attachable to the breast to enable at least part of the breast to be subjected to an under-pressure created following compression of the container. The container is compressible along a compression axis (CD) to enable the under-pressure to be created. The container is partly delimited by a wall portion (11A), e.g. a rigid wall portion, that is coupled to a resiliently deformable assembly (11B; 12, 13, 14, 15). The container is compressible by displacement of the wall portion along the compression axis against a restoring force of the resiliently deformable assembly that is deformed during the displacement of the wall portion.

## Description

### FIELD OF THE INVENTION

The invention relates to a breast milk collector, also known as a passive milk collector, and a method of interfacing the breast milk collector with a breast.

### BACKGROUND OF THE INVENTION

The best source of nutrition for a baby is human milk given by a breastfeeding mother. The World Health Organization recommends to breast feed for at least one year, preferably longer. However, mothers often go back to work after only several weeks or months. To provide the best nutrition to their babies, mothers express milk using a breast pump. The expressed milk can be stored and given to the baby at a later stage by the mother and/or by somebody else.

Recently, there is a trend towards wearable passive breast milk collectors that collect leaking breast milk. Such wearable passive breast milk collector can be used with the baby present. When the baby starts to suckle at a breast, nipple stimulation may cause the milk ejection reflex (MER) at both breasts. The breast milk collector then collects milk from the mother's other breast via a passive continuous negative pressure, in other words a partial vacuum.

Such breast milk collectors can be formed as compressible milk containers which can be pressed inwards towards the mother's body to provide a holding pressure and a base vacuum for enhancing milk extraction. The breast milk collectors available on the market tend to have a soft silicone shell that can be compressed and positioned on the breast by the user. The shape of the shell and the elastic characteristics of the material may determine the pressure profile/behavior of the breast milk collector during expression. In particular, the material properties, wall thickness and shape of the silicone shell may determine the shell's spring constant that, in combination with the effective surface on which the spring force is acting, is responsible for the under-pressure generated by the breast milk collector.

### SUMMARY OF THE INVENTION

Laboratory analysis of currently available breast milk collectors revealed that the under-pressure achievable when using such breast milk collectors tends not to depend much on how strongly the user compresses the soft shell, e.g. when positioning the shell on the breast and compressing the soft shell to provide the under-pressure, for the majority of the working windows of such devices. Moreover, the manner in which the soft shell of the currently available breast milk collectors is compressible was found to pose difficulties in terms of providing under-pressure adjustment that could be beneficial to the user, for example adjustment that enables the user to retain or revert to an initial under-pressure provided at the start of milk collection. It is noted in this respect that breast milk being received in the breast milk collector tends to cause progressive loss of under-pressure during use.

Therefore, the user may not be able to steer or adapt the pressure profile behavior of currently available breast milk collectors. It would be desirable to design a breast milk collector in a way that facilitates under-pressure adjustment, particularly noting that users may have different sensitivities and preferences in relation to the under-pressure to which their breast is subjected when using a breast milk collector.

The invention is defined by the claims.

According to examples in accordance with an aspect of the invention, there is provided a breast milk collector for collecting milk from a mother's breast, the breast milk collector comprising a container, the container being partly delimited by a wall portion, the breast milk collector being attachable to the breast to enable at least part of the breast to be subjected to an under-pressure created following displacement of the wall portion to compress the container, wherein the wall portion is coupled to a resiliently deformable assembly, the wall portion being displaceable along a compression axis to deform the resiliently deformable assembly and compress the container, wherein the wall portion is less deformable than the resiliently deformable assembly along the compression axis by force directed along the compression axis.

The breast milk collector can be regarded as a passive breast milk collector that receives milk while the mother's other breast is being actively stimulated by a variable/alternating pressure provided by a feeding infant or a breast pump.

An under-pressure, e.g. a partial vacuum, can be generated in the breast milk collector's container by compressing the container via displacement of the container's wall portion and associated deformation of the resiliently deformable assembly coupled to the wall portion. The under-pressure can encourage milk ejection from the breast interfaced with the breast milk collector.

This compression of the container leads to generation of the under-pressure, e.g. a partial vacuum, in the container. As explained in more detail herein below, a restoring force acts to bring the container back to its normal/uncompressed shape in relation with the volume of air that is pushed out during the compression. Therefore, there is a shortage of air and hence the under-pressure in the container.

The spring force, in other words restoring force, of the resiliently deformable assembly may balance the force resulting from the gas pressure difference between the inside of the compressed container and the outside of the compressed container, in other words atmospheric pressure. This means that the under-pressure achievable in the compressed container is partly influenced by the spring force of the resiliently deformable assembly.

The present invention is partly based on the realization that the under-pressure within the container can be better controlled by employing a wall portion that, when subjected to a force directed along the compression axis, deforms less along the compression axis than the resiliently deformable assembly's deformation along the compression axis. In this way, the under-pressure can be primarily determinable by selecting the spring force of the resiliently deformable assembly and the area of the less deformable, and in some embodiments rigid, wall portion that is acted upon by the resiliently deformable assembly.

This force may be applied, e.g. by the user's finger(s), along the compression axis to cause compression of the container.

In some embodiments, the resiliently deformable assembly is configurable to adjust a spring force of the resiliently deformable assembly that resists displacement of the wall portion along the compression axis. Such spring force adjustment may enable adjustment of the under-pressure in the compressed container. The spring force adjustment may enable a desired under-pressure to be maintained in the compressed container, for example to compensate for reduction of the gas pressure difference between the inside and the outside of compressed chamber resulting from milk ejection from the breast during use.

Alternatively or in addition to the spring force adjustment, the wall portion may be configurable to adjust a wall portion area of the wall portion acted on by the resiliently deformable assembly. The under-pressure in the compressed container may be approximated by the spring force divided by the wall portion area acted on by the spring force. Hence adjusting the wall portion area may provide a way of adjusting the under-pressure.

In some embodiments, the breast milk collector comprises a rigid chassis for supporting the container.

In some embodiments, the rigid chassis, e.g. hard shell or frame, is configured to support the container and a detachable milk collection container, e.g. a disposable milk bag. Alternatively, the container itself may be configured as a milk collection container, such as a milk collection bottle or a disposable milk bag.

In some embodiments, the container is detachable from the rigid chassis, e.g. from the hard shell or frame.

This detachability may facilitate cleaning of the container and/or the rigid chassis. Such detachability may also facilitate storing of the breast milk collected in the container in embodiments in which the container itself is configured as a milk collection container.

In some embodiments, the breast milk collector comprises a lever coupled to the chassis, with the lever being arranged to exert a resisting force on the wall portion that resists displacement of the wall portion along the compression axis. The rigidity of the chassis, e.g. hard shell or frame, may enable the lever to exert the resisting force on the wall portion.

The lever may, for example, be pivotably coupled to the chassis, with the resisting force being exerted on the wall portion via pivoting of the lever towards, e.g. against, the wall portion.

In some embodiments, the lever is arranged to pull or push the wall portion towards a stopper that stops displacement of the wall portion during expansion of the container. In such embodiments, the lever may be moved, e.g. bent, so as to compress the container, thereby forcing air from the container. When the lever is released, stress, e.g. bending stress, in the lever may pull at the wall portion.

Initially, the lever may pull at a relatively large area of the wall portion, due to the stiffness of the less deformable wall portion retaining its shape during compression of the container.

However, during collection of milk, the container may progressively expand, thereby relieving stress in the lever. When the wall portion is stopped from moving further by the stopper, e.g. a portion of the chassis, an effective area at which the lever is pulling may reduce. Together with the reduced lever stress, which reduces the pulling force, a relatively constant vacuum pressure can be maintained within the container.

To this end, the reduction in pulling force may be balanced with the reduction in effective pulling area, until the displacement is stopped.

A mother-specific pressure can be obtained by using different levers, levers of different materials or thickness, or by using leaf springs. Correct expansion of the container, e.g. via unfolding of a part of the container, can be realized by, for instance, a thickness variation of the material forming the container.

In some embodiments, the resiliently deformable assembly comprises a spring.

Such a spring may facilitate control over the under-pressure in the compressed container, since the spring force of the spring may be selectable, and in some embodiments selectable by the user during use of the breast milk collector.

In some embodiments, the spring comprises, e.g. is, a constant force spring.

The constant spring force provided by such a constant force spring may assist to control the under-pressure in the compressed container.

The wall portion may be displaceable along the compression axis from an initial position corresponding to an uncompressed state of the container to a final position reached at maximum displacement of the wall portion.

The spring, e.g. the constant force spring, may be configured such that the spring's spring force when the wall portion is in the initial position is at least 80%, preferably at least 90%, of the spring's spring force when the wall portion is in the final position. This relatively constant spring force may enhance control over the under-pressure in the compressed container.

In some embodiments, the spring, e.g. the constant force spring, is a torsion spring.

In some embodiments, the spring, e.g. the constant force and/or torsion spring, is between the chassis and the lever, with the spring being arranged to bias the lever into exerting the force on the wall portion.

It is noted that the above-mentioned torsion spring may be particularly suitable for biasing the lever, e.g. the lever pivotably mounted to the chassis, into exerting the resisting force on the wall portion.

In some embodiments, the spring is configurable to enable selection of the spring's spring force. Selection of the spring's spring force may enable relatively straightforward adjustment of the under-pressure in the compressed container.

In such embodiments, the spring's spring force may be adjustable, for instance, by winding and unwinding a strip, e.g. steel strip, of a spiral wound torsion spring.

The breast milk collector may include an adjustment mechanism configured to enable a user of the breast milk collector to select the spring's spring force, for example by the adjustment mechanism being configured to enable a user to wind and unwind the strip of the spiral wound torsion spring.

In some embodiments, the adjustment mechanism is configured to provide self-locking selection of the spring's spring force. Thus, the adjustment mechanism may enable the user, in a single action, to select the spring's spring force and for the selected spring force to be locked until the adjustment mechanism is subsequently used to re-select the spring force.

In some embodiments, the adjustment mechanism comprises a worm wheel that is rotatable by the user in a first direction to increase the spring's spring force and rotatable in a second direction opposite to the first direction to decrease the spring's spring force. Such a worm wheel may thus enable the user to select the spring's spring force by turning of the worm wheel, with the selected spring force being locked by the worm wheel once the turning action is complete. The selected spring force is locked until the worm wheel is subsequently turned to re-select the spring force.

In some embodiments, the resiliently deformable assembly comprises a resiliently deformable wall section of the container. Thus, the wall section, e.g. sidewall section, of the container may be designed so as to contribute to the resilient deformability of the resiliently deformable assembly.

The wall section may, for instance, comprise a concertinaed wall section.

In some embodiments, the container is defined by a rigid wall portion for arranging opposite the breast and a concertinaed sidewall section that, in use, extends from the rigid wall portion towards the breast. The container in such embodiments can be regarded, for instance, as a bellows-type container.

In some embodiments, part of the container is switchable between being added to the wall portion, and thereby increasing the wall portion's area acted on by the resiliently deformable assembly, and being removed from the wall portion, and thereby decreasing the wall portion's area acted on by the resiliently deformable assembly. This switching, e.g. via unfolding and folding of the part of the container, may enable relatively straightforward adjustment of the wall portion area being acted upon by the resiliently deformable assembly, and thus facile adjustment of the under-pressure in the compressed container.

In some embodiments, the breast milk collector comprises one or more reinforcement elements arranged to retain the shape of the part of the container when the part of the container is added to the wall portion. Thus, the part of the container may be secured in a switched state, e.g. an unfolded state, in which the part of the container is added to/included in the wall portion that is acted upon by the resiliently deformable assembly.

According to another aspect there is provided a method of interfacing a breast milk collector with a mother's breast, the breast milk collector comprising a container that is partly delimited by a wall portion coupled to a resiliently deformable assembly, the method comprising: attaching the breast milk collector to the breast; and applying a force to the wall portion along a compression axis to compress the container and thereby provide an under-pressure in the container, the force causing less deformation of the wall portion along the compression axis than the resiliently deformable assembly's deformation along the compression axis.

The method may be a method of interfacing a breast milk collector according to any of the embodiments described herein with a mother's breast.

The method may not include expression of milk from the mother's breast into the breast milk collector.

In some embodiments, the method comprises adjusting a spring force of the resiliently deformable assembly to adjust the under-pressure.

Alternatively or additionally, the method comprises adjusting a wall portion area of the wall portion acted on by the resiliently deformable assembly to adjust the under-pressure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1A provides a schematic cross-sectional view of a breast milk collector according to an example during compression of a container of the breast milk collector;
FIG. 1B provides a schematic cross-sectional view of the breast milk collector shown in FIG. 1A with the container being in an expanded state;
FIG. 1C provides an exterior view of the breast milk collector shown in FIGs. 1A and 1B;
FIG. 2 provides under-pressure profiles with constant flow per spring force of a breast milk collector's resiliently deformable assembly;
FIGs. 3A and 3B provide views of a breast milk collector according to another example, with the breast milk collector being in a first configuration;
FIGs. 3C and 3D provide views of the breast milk collector shown in FIGs. 3A and 3B in a second configuration different from the first configuration;
FIG. 4A provides a schematic cross-sectional view of a breast milk collector according to a further example, with the breast milk collector's container being in a compressed state; and
FIG. 4B provides a schematic cross-sectional view of the breast milk collector shown in FIG. 4A, with the breast milk collector's container being in an expanded state.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a passive breast milk collector for collecting milk from a mother's breast. The breast milk collector comprises a container. The breast milk collector is attachable to the breast to enable at least part of the breast to be subjected to an under-pressure created following compression of the container. The container is compressible along a compression axis to enable the under-pressure to be created. The container is partly delimited by a wall portion, e.g. a rigid wall portion, that is coupled to a resiliently deformable assembly. The container is compressible by displacement of the wall portion along the compression axis against a restoring force of the resiliently deformable assembly that is deformed during the displacement of the wall portion.

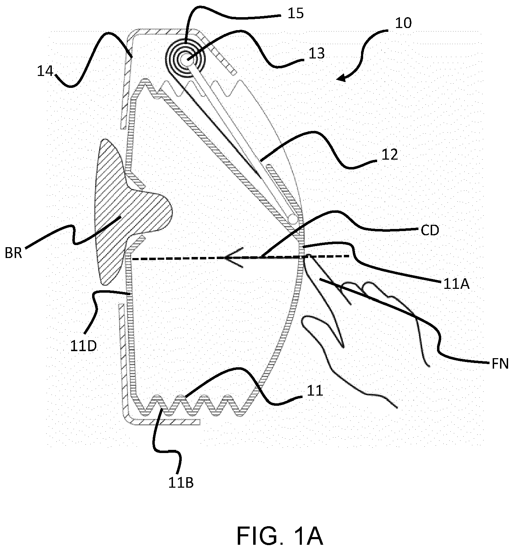

FIGs. 1A to 1C schematically depict a breast milk collector 10 according to an example. The breast milk collector 10 comprises a container 11 attachable to a breast BR of a user to enable at least part of the breast BR to be subjected to an under-pressure provided within the container 11 when the container 11 is compressed.

The at least part of the breast BR may be received in the container 11, or the container 11 may be in fluid communication with a cavity in which the at least part of the breast BR is receivable in order for it to be subjected to the under-pressure provided within the container 11.

The container 11 is compressible along a compression axis CD to enable the under-pressure to be provided. In particular, the container 11 is partly delimited by a wall portion 11A to which a force can be applied along the compression axis CD, e.g. by the user's finger(s) FN, in order to compress the container 11.

This compression of the container 11 leads to generation of the under-pressure, e.g. a partial vacuum, in the container 11. As explained in more detail herein below, a restoring force acts to bring the container 11 back to its normal/uncompressed shape in relation with the volume of air that is pushed out during the compression. Therefore, there is a shortage of air and hence the under-pressure in the container 11. The under-pressure can encourage milk ejection from the breast BR interfaced with the breast milk collector 10.

The breast milk collector 10 preferably includes a valve which allows air to flow out during compression of the container 11 but prevents air from flowing into the container 11.

More generally, the wall portion 11A is coupled to a resiliently deformable assembly 11B; 12, 13, 14, 15. The wall portion 11A is less deformable than the resiliently deformable assembly 11B; 12, 13, 14, 15 along the compression axis CD by force directed along the compression axis, e.g. the force applied to the wall portion 11A along the compression axis CD to compress the container 11.

For example, the wall portion 11A may be stiffer than the resiliently deformable assembly 11B; 12, 13, 14, 15 so that the resiliently deformable assembly 11B; 12, 13, 14, 15 deforms preferentially with respect to the resiliently deformable assembly 11B; 12, 13, 14, 15 when the wall portion 11A is subjected to the force along the compression axis CD.

The under-pressure within the container 11 can be better controlled by employing such a wall portion 11A that, when subjected to the force along the compression axis CD, e.g. for compressing the container 11, deforms less along the compression axis CD than the resiliently deformable assembly's 11B; 12, 13, 14, 15 deformation along the compression axis CD. In this way, the under-pressure can be primarily determinable by selecting the spring force of the resiliently deformable assembly 11B; 12, 13, 14, 15 and the area of the less deformable, and in some embodiments rigid, wall portion 11A that is acted upon by the resiliently deformable assembly 11B; 12, 13, 14, 15.

The wall portion's 11A deformability, e.g. compressibility, along the compression axis CD can be made smaller than that of the resiliently deformable assembly 11B; 12, 13, 14, 15 at least partly by selection of material and/or thickness of the wall portion 11A.

The resiliently deformable assembly 11B; 12, 13, 14, 15 may be configured in any suitable manner. In some embodiments, such as shown in FIGs. 1A to 1C, the resiliently deformable assembly 11B; 12, 13, 14, 15 comprises, and in some cases may be defined by, a resiliently deformable wall section 11B of the container 11. Thus, the wall section 11B, e.g. sidewall section 11B, of the container 11 may be designed so as to contribute to, or in some cases provide, the resilient deformability of the resiliently deformable assembly 11B; 12, 13, 14, 15.

The wall section 11B may, for instance, comprise a concertinaed wall section 11B. In some embodiments, such as shown in FIGs. 1A to 1C, the container 11 is defined by a rigid wall portion 11A for arranging opposite the breast BR and a concertinaed sidewall section 11B that, in use, extends from the rigid wall portion 11A towards the breast BR. The container 11 in such embodiments can be regarded, for instance, as a bellows-type container 11.

In some embodiments, the material of the wall section 11B is the same as that of the wall portion 11A, but with the configuration, e.g. concertinaed shape, of the wall section 11B, for example together with a greater thickness of the wall portion 11A, ensuring that the wall portion 11A is less deformable along the compression axis CD than the wall section 11B-comprising resiliently deformable assembly 11B; 12, 13, 14, 15.

In other embodiments, the material of the wall section 11B is different from, e.g. more compressible along the compression axis CD than, the material of the wall portion 11A.

Materials for the wall portion 11A and the wall section 11B can be selected from, for example, polymeric materials, such as plastics and/or elastomers. Particular mention is made of silicone as a material for forming the wall portion 11A and/or the wall section 11B.

As an alternative or in addition to the resiliently deformable assembly 11B; 12, 13, 14, 15 comprising the resiliently deformable wall section 11B, a spring 15 may be included in the resiliently deformable assembly 11B; 12, 13, 14, 15 for resisting displacement of the wall portion 11A along the compression axis CD. Such a spring 15 may facilitate control over the under-pressure in the compressed container 11, since the spring force of the spring 15 may be selectable, for instance selectable by the user during use of the breast milk collector 10, as explained in more detail herein below.

The spring 15 can have any suitable design and can be arranged in any suitable manner to resist displacement of the wall portion 11A along the compression axis CD. In some embodiments, the spring 15 comprises, e.g. is, a torsion spring.

In more general terms, the spring force, in other words restoring force, of the resiliently deformable assembly 11B; 12, 13, 14, 15 may balance the force resulting from the gas pressure difference between the inside of the compressed container 11 and the outside of the compressed container 11, in other words atmospheric pressure. This means that the under-pressure achievable in the compressed container 11 is partly influenced by the spring force of the resiliently deformable assembly 11B; 12, 13, 14, 15.

In some embodiments, such as shown in FIGs. 1A to 1C, the spring 15 comprises, e.g. is, a constant force spring, such as a constant torque spring 15, that exerts a consistent force, or at least a substantially consistent force, over its range of motion. The constant spring force provided by such a constant force spring 15 may assist to control the under-pressure in the compressed container 11.

In some embodiments, such as shown in FIGs. 1A to 1C, the constant force spring 15 is a spiral wound torsion spring 15, for example a steel strip spring 15.

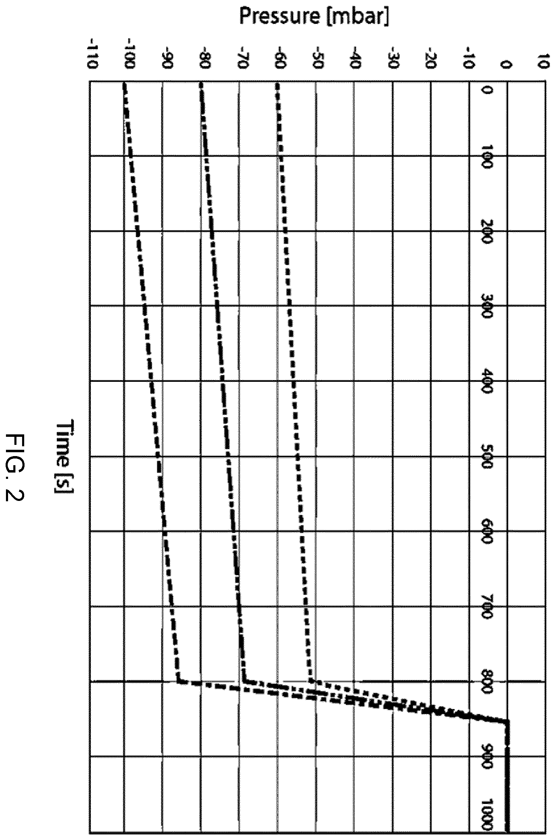

FIG. 2 provides under-pressure profiles with constant inflow of milk per spring force of the breast milk collector's 10 resiliently deformable assembly 11B; 12, 13, 14, 15. FIG. 2 demonstrates that adjustment of the spring force enables the under-pressure to be adjusted, in particular in a way that may be beneficial to the user. In this respect, FIG. 2 shows that spring force adjustment may enable the user to revert to an initial under-pressure provided at the start of milk collection.

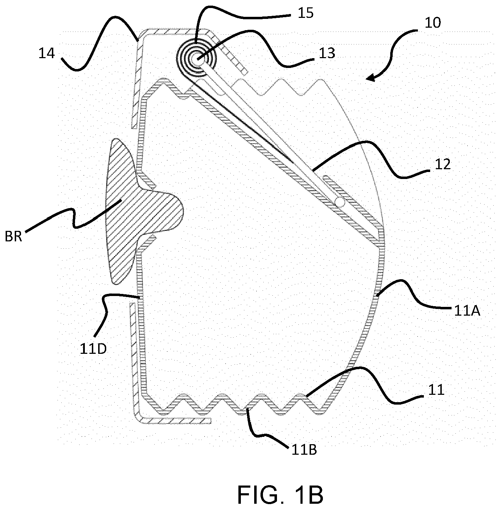

It is noted that the wall portion 11A may be displaceable along the compression axis CD from an initial position, as shown in FIG. 1B, corresponding to an uncompressed state of the container 11 to a final position reached at maximum displacement of the wall portion 11A.

The spring 15, e.g. the constant force spring 15, may be configured such that the spring's 15 spring force when the wall portion 11A is in the initial position is at least 80%, preferably at least 90%, of the spring's 15 spring force when the wall portion 11A is in the compressed position. This relatively constant spring force may enhance control over the under-pressure in the compressed container 11.

In some embodiments, the resiliently deformable assembly 11B; 12, 13, 14, 15 is configurable to adjust a spring force of the resiliently deformable assembly 11B; 12, 13, 14, 15 that resists displacement of the wall portion 11A along the compression axis CD. Such spring force adjustment may enable adjustment of the under-pressure in the compressed container 11.

Adjustment of the spring force of the resiliently deformable assembly 11B; 12, 13, 14, 15 may be implemented in any suitable manner. In some embodiments, such as shown in FIGs. 1A to 1C, the spring 15 is configurable to enable selection of the spring's 15 spring force. Selection of the spring's 15 spring force may enable relatively straightforward adjustment of the under-pressure in the compressed container 11.

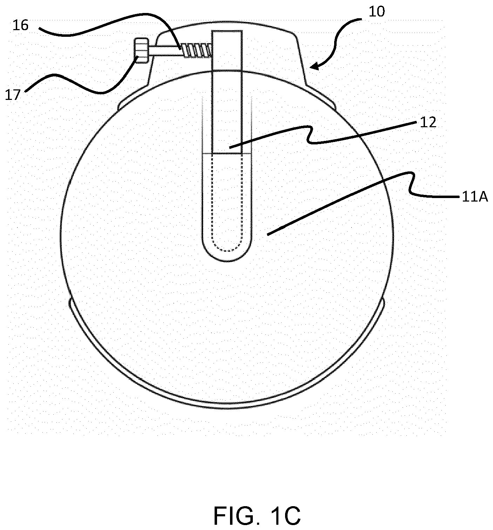

In such embodiments, the spring's 15 spring force may be adjustable by winding and unwinding a strip, e.g. steel strip, of a spiral wound torsion spring 15. Referring in particular to FIG. 1C, the breast milk collector 10 may include an adjustment mechanism 16 configured to enable a user of the breast milk collector 10 to select the spring's 15 spring force. In such embodiments, the adjustment mechanism 16 may be configured to enable the user to wind and unwind the strip of the spiral wound torsion spring 15, e.g. by turning a knob 17 included in the adjustment mechanism 16.

In some embodiments, the adjustment mechanism 16 is configured to provide self-locking selection of the spring's 15 spring force. Thus, the adjustment mechanism 16 may enable the user, in a single action, to select the spring's 15 spring force and for the selected spring force to be locked until the adjustment mechanism 16 is subsequently used to re-select the spring force.

In some embodiments, such as shown in FIG. 1C, the adjustment mechanism 16 comprises a worm wheel that is rotatable by the user in a first direction to increase the spring's 15 spring force and rotatable in a second direction opposite to the first direction to decrease the spring's 15 spring force. Such a worm wheel may thus enable the user to select the spring's 15 spring force by turning of the worm wheel, with the selected spring force being locked by the worm wheel once the turning action is complete. The selected spring force is locked until the worm wheel is subsequently turned to re-select the spring force.

In some embodiments, such as shown in FIGs. 1A to 1C, the breast milk collector 10 comprises a rigid chassis 14 for supporting the container 11. For example, the container 11 itself may be relatively flexible and elastic, and fit within the chassis 14, e.g. a chassis 14 in the form of a hard shell or frame.

The chassis 14 may be formed from any suitable rigid material, such as a rigid plastic material, e.g. polypropylene.

The rigid chassis 14, e.g. hard shell or frame, may be configured to support the container 11 and a detachable milk collection container, e.g. a disposable milk bag. Alternatively, the container 11 itself may be configured as a milk collection container, such as a milk collection bottle or a disposable milk bag.

In some embodiments, the container 11 is detachable from the rigid chassis 14, e.g. from the hard shell or frame. This detachability may facilitate cleaning of the container 11 and/or the rigid chassis 14. Such detachability may also assist with storing of the breast milk collected in the container 11 in embodiments in which the container 11 itself is configured as a milk collection container.

In some embodiments, such as shown in FIGs. 1A to 1C, the breast milk collector 10 comprises a lever 12 coupled to the chassis 14, with the lever 12 being arranged to exert a resisting force on the wall portion 11A that resists displacement of the wall portion 11A along the compression axis CD. The rigidity of the chassis 14, e.g. hard shell or frame, may enable the lever 12 to exert the resisting force on the wall portion 11A.

The lever 12 may be arranged to push and pull at the container 11 through a gap defined in the chassis 14, e.g. hard shell.

The lever 12 can be formed from any suitable rigid material. In some embodiments, the lever 12 is formed from a plastic material, e.g. polypropylene.

As best shown in FIG. 1A, the lever 12 may be pivotably coupled to the chassis 14, with the resisting force being exerted on the wall portion 11A via pivoting of the lever 12 towards, e.g. against, the wall portion 11A. To this end, the breast milk collector 10 may include a pivot coupling 13, e.g. a hinge, between the lever 12 and the chassis 14.

In some embodiments, the spring 15, e.g. the constant force spring 15 and/or the torsion spring 15, is between the chassis 14 and the lever 12, with the spring 15 being arranged to bias the lever 15 into exerting the resisting force on the wall portion 11A. It is noted that the above-mentioned torsion spring 15 may be particularly suitable for biasing the lever 12 pivotably mounted to the chassis 14 into exerting the resisting force on the wall portion 11A.

In a non-limiting illustrative example, and with reference to FIGs. 1A to 1C, the container 11 may be a flexible container 11 with a relatively stiff top wall portion 11A and a relatively stiff bottom wall portion 11D, and a flexible sidewall section 11B extending between the top wall portion 11A and the bottom wall portion 11D. It is reiterated in relation to this example that the container 11 preferably has a valve which allows air to flow out of the container 11 but prevents air from flowing into the container 11.

In this non-limiting example, the stiff lever 12 is connected with the hinge 13 to the chassis 14, e.g. hard shell, which holds the container 11, with a constant force torsion spring 15 being attached to the hinge 13 and the lever 12. This spring 15 may be pre-stressed by, for example, use of the worm wheel mechanism 16. The lever 12 can be pressed inwards. The tension of the torsion spring 15 can be changed by the worm wheel adjustor 17, e.g. knob. With continued reference to FIGs. 1A to 1C, the top wall portion 11A of the container 11 is attached to the lever 12. When the lever 12 is pressed it compresses the container 11. When the lever 12 is released, the constant force torsion spring 15 applies a constant force/torque to the lever 12 and the top wall portion 11A of the container 11.

As the container 11 in this non-limiting example has a stiff top wall portion 11A, this force is transferred to this top wall portion 11A, which may provide a relatively constant/continuous under-pressure in the container 11. The under-pressure may keep the container 11 attached to the nipple of the breast BR, with no air being admitted into the container 11. When milk starts to flow into the container 11, its volume is slowly filled. However, the vacuum of the container 11 may remain relatively constant due to the constant pull of the lever 12. The vacuum may reduce when the lever 12 reaches its end-stop and is no longer able to pull. The lever 12 can be pushed multiple times.

As milk flow and comfort can be optimal at a certain mother-specific pressure and might even change during many months of breast feeding, the pre-stress of the spring 15 can be changed. Releasing or increasing this pre-stress can be done in this non-limiting example via the worm wheel mechanism 16. In more general terms, the under-pressure may be relatively constant and adaptable due to the lever 12 and spring 15 mechanism. This may assist to optimize comfort and milk collection performance.

It is noted at this point that the design of the wall portion 11A is not particularly limited provided that the wall portion 11A is less deformable along the compression axis CD than the resiliently deformable assembly 11B; 12, 13, 14, 15 coupled to the wall portion 11A. In some embodiments (not shown), the wall portion 11A comprises, e.g. is defined by, an end of a plunger that moves along a barrel. In such embodiments, the end of the plunger may be less deformable along the compression axis CD than the resiliently deformable assembly 11B; 12, 13, 14, 15, e.g. spring 15 or lever-spring 12, 15 arrangement, that resists movement of the end of the plunger along the compression axis CD.

In some embodiments, the wall portion 11A is configurable to adjust a wall portion area of the wall portion acted on by the resiliently deformable assembly 11B; 12, 13, 14, 15. The under-pressure in the compressed container 11 may be approximated by the spring force divided by the wall portion area acted on by the spring force. Hence adjusting the wall portion area may provide a way of adjusting the under-pressure, for example as an alternative or in addition to the spring force of the resiliently deformable assembly 11B; 12, 13, 14, 15 being adjustable.

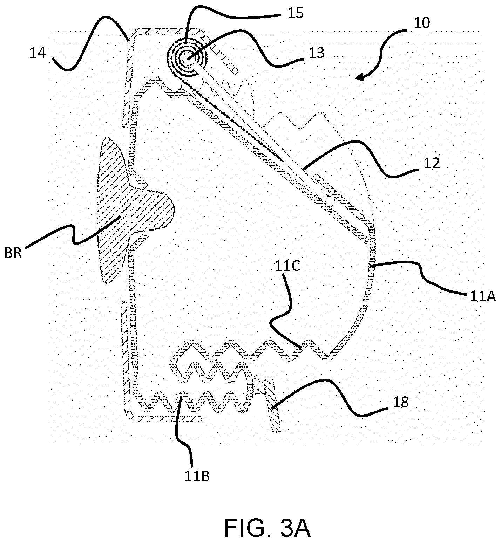

Such wall portion area adjustment may be implemented in any suitable manner. In some embodiments, and referring to FIGs. 3A to 3D, part 11C of the container 11 is switchable between being added to the wall portion 11A, and thereby increasing the wall portion area acted on by the resiliently deformable assembly 11B; 12, 13, 14, 15, and being removed from the wall portion 11A, and thereby decreasing the wall portion area acted on by the resiliently deformable assembly 11B; 12, 13, 14, 15.

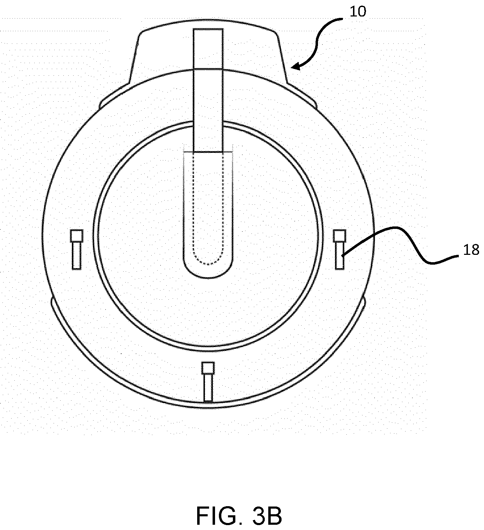

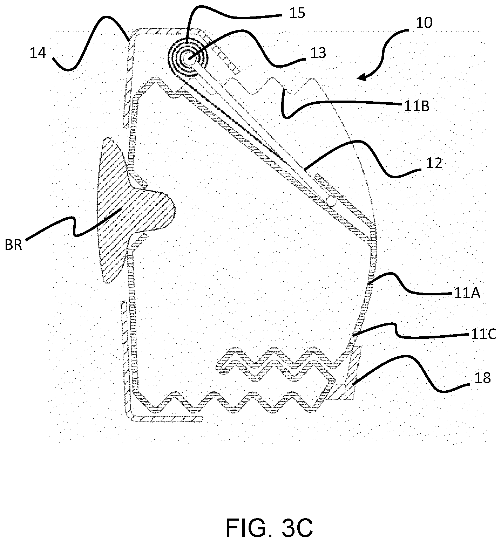

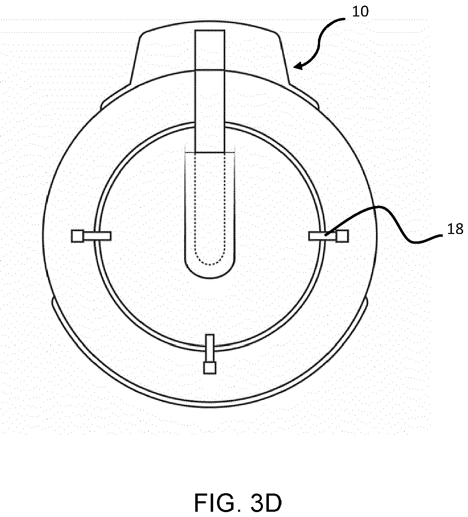

In the configuration shown in FIGs. 3A and 3B, the part 11C of the container 11 is not included in the wall portion area acted on by the resiliently deformable assembly 11B; 12, 13, 14, 15, whereas in the configuration shown in FIGs. 3C and 3D, the part 11C of the container 11 is included in the wall portion area acted on by the resiliently deformable assembly 11B; 12, 13, 14, 15. The increased wall portion area in the configuration shown in FIGs. 3C and 3D may decrease the under-pressure in the container 11. This switching, e.g. via unfolding and folding of the part 11C of the container 11, may enable relatively straightforward adjustment of the wall portion area being acted upon by the resiliently deformable assembly 11B; 12, 13, 14, 15, and thus facile adjustment of the under-pressure in the compressed container 11.

It is noted that the switching of the part 11C of the container 11 may cause volume change of the container 11, for example a volume change of around 10% or more than 10%. It is noted that the initial volume may be most important for the duration of the milk expression session. Less volume displaced may mean less milk can be expressed.

In some embodiments, such as shown in FIGs. 3A to 3D, the breast milk collector 10 comprises one or more reinforcement elements 18 arranged to retain the shape of the part 11C of the container 11 when the part 11C of the container 11 is added to the wall portion 11A. Thus, the part 11C of the container 11 may be secured in a switched state, e.g. an unfolded state, in which the part 11C of the container 11 is added to/included in the wall portion 11A that is acted upon by the resiliently deformable assembly 11B; 12, 13, 14, 15.

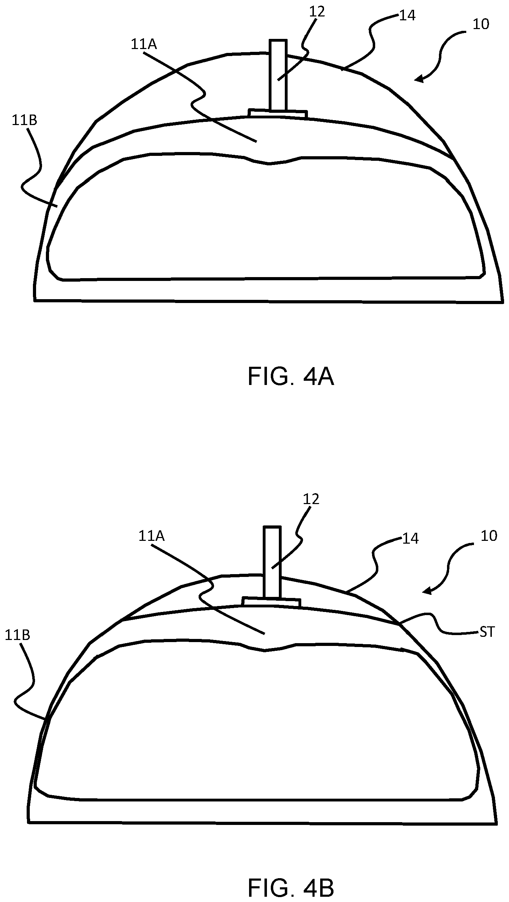

In some embodiments, such as shown in FIGs. 4A and 4B, the lever 12 is arranged to pull the wall portion 11A towards a stopper ST that stops displacement of the wall portion 11A during expansion of the container 11. In such embodiments, the lever 12 may be moved, e.g. bent, so as to compress the container 11, thereby forcing air from the container 11. When the lever 12 is released, stress, e.g. bending stress, in the lever 12 may pull at the wall portion 11A.

Initially, the lever 12 may pull at a relatively large area of the wall portion 11A, due to the stiffness of the less deformable wall portion 11A retaining its shape during compression of the container 11. However, during collection of milk, the container 11 may progressively expand, thereby relieving stress in the lever 12. When the wall portion 11A is stopped from moving further by the stopper ST, e.g. a portion of the chassis 14, an effective area at which the lever 12 is pulling may reduce. Together with the reduced lever stress, which reduces the pulling force, a relatively constant vacuum pressure can be maintained within the container 11. To this end, the reduction in pulling force may be balanced with the reduction in effective pulling area, until the displacement is stopped.

A mother-specific pressure can be obtained by using different levers 12, levers 12 of different materials or thickness, or by using leaf springs and/or springs that are connected or not connected to each other or to the wall portion 11A. Correct expansion of the container 11, e.g. via unfolding of a part of the container 11, can be realized, for example, by a thickness variation of the material forming the container 11.

The present disclosure also provides a method of interfacing a breast milk collector 10 with a mother's breast BR. The breast milk collector 10 comprises a container 11 that is partly delimited by a wall portion 11A coupled to a resiliently deformable assembly 11B; 12, 13, 14, 15. The breast milk collector 10 may be according to any of the embodiments described herein, for example with reference to FIGs. 1A to 1C, 3A to 3D, 4A and 4B. The method comprises attaching the breast milk collector 10, e.g. the container 11 thereof, to the breast BR, and applying a force, e.g. with the user's finger(s) FN, to the wall portion 11A along a compression axis CD to compress the container 11 and thereby provide an under-pressure in the container 11. The force causes less deformation of the wall portion 11A along the compression axis CD than the resiliently deformable assembly's 11B; 12, 13, 14, 15 deformation along the compression axis CD, as previously described.

In some embodiments, the method comprises adjusting a spring force of the resiliently deformable assembly 11B; 12, 13, 14, 15 to adjust the under-pressure, for example by selecting the spring force of a configurable spring 15 included in the resiliently deformable assembly 11B; 12, 13, 14, 15.

Alternatively or additionally, the method may comprise adjusting a wall portion area of the wall portion 11A acted on by the resiliently deformable assembly 11B; 12, 13, 14, 15 to adjust the under-pressure, for example by switching a part 11C of the container 11 between being added to the wall portion 11A, and thereby increasing the wall portion area acted on by the resiliently deformable assembly 11B; 12, 13, 14, 15, and being removed from the wall portion 11A, and thereby decreasing the wall portion area acted on by the resiliently deformable assembly 11B; 12, 13, 14, 15.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast milk collector (10) for collecting milk from a mother's breast, the breast milk collector comprising a container (11), the container being partly delimited by a wall portion (11A), the breast milk collector being attachable to the breast to enable at least part of the breast to be subjected to an under-pressure created following displacement of the wall portion to compress the container, wherein the wall portion (11A) is coupled to a resiliently deformable assembly (11B; 12, 13, 14, 15), the wall portion being displaceable along a compression axis (CD) to deform the resiliently deformable assembly and compress the container, wherein the wall portion is less deformable than the resiliently deformable assembly along the compression axis by force directed along the compression axis.

2. The breast milk collector (10) according to claim 1, wherein the resiliently deformable assembly (11B; 12, 13, 14, 15) is configurable to adjust a spring force of the resiliently deformable assembly that resists displacement of the wall portion (11A) along the compression axis (CD).

3. The breast milk collector (10) according to claim 1 or claim 2, wherein the wall portion (11A) is configurable to adjust a wall portion area of the wall portion acted on by the resiliently deformable assembly (11B; 12, 13, 14, 15).

4. The breast milk collector (10) according to any one of claims 1 to 3, comprising a rigid chassis (14) for supporting the container (11), and a lever (12) coupled to the chassis, the lever being arranged to exert a resisting force on the wall portion (11A) that resists displacement of the wall portion along the compression axis (CD).

5. The breast milk collector (10) according to claim 4, wherein the lever (12) is arranged to pull or push the wall portion (11A) towards a stopper (ST) that stops displacement of the wall portion during expansion of the container (11).

6. The breast milk collector (10) according to claim 4 or claim 5, wherein the resiliently deformable assembly (11B; 12, 13, 14, 15) comprises a spring (15) between the chassis (14) and the lever (12), the spring being arranged to bias the lever (12) into exerting the resisting force on the wall portion (11A).

7. The breast milk collector (10) according to claim 6, wherein the spring (15) comprises a constant force spring; and/or wherein the spring comprises a torsion spring.

8. The breast milk collector (10) according to claim 6 or claim 7, wherein the wall portion (11A) is displaceable along the compression axis (CD) from an initial position corresponding to an uncompressed state of the container (11) to a final position reached at maximum displacement of the wall portion, the spring (15) being configured such that the spring's spring force when the wall portion is in the initial position is at least 80%, preferably at least 90%, of the spring's spring force when the wall portion is in the final position.

9. The breast milk collector (10) according to any one of claims 6 to 8, wherein the spring (15) is configurable to enable selection of the spring's spring force.

10. The breast milk collector (10) according to claim 9, comprising an adjustment mechanism (16) configured to enable a user of the breast milk collector to select the spring's (15) spring force.

11. The breast milk collector (10) according to claim 10, wherein the adjustment mechanism (16) is configured to provide self-locking selection of the spring's (15) spring force; optionally wherein the adjustment mechanism comprises a worm wheel that is rotatable by the user in a first direction to increase the spring's spring force and rotatable in a second direction opposite to the first direction to decrease the spring's spring force.

12. The breast milk collector (10) according to any one of claims 1 to 11, wherein the resiliently deformable assembly (11B; 12, 13, 14, 15) comprises a resiliently deformable wall section (11B) of the container; optionally wherein the wall section comprises a concertinaed wall section.

13. The breast milk collector (10) according to any one of claims 1 to 12, wherein part (11C) of the container (11) is switchable between being added to the wall portion (11A), and thereby increasing the wall portion's area acted on by the resiliently deformable assembly (11B; 12, 13, 14, 15), and being removed from the wall portion, and thereby decreasing the wall portion's area acted on by the resiliently deformable assembly.

14. The breast milk collector (10) according to any one of claims 1 to 13, wherein the container (11) is configured as a milk collection container.

15. A method of interfacing a breast milk collector (10) with a mother's breast, the breast milk collector comprising a container (11) that is partly delimited by a wall portion (11A) coupled to a resiliently deformable assembly (11B; 12, 13, 14, 15), the method comprising:
attaching the breast milk collector to the breast; and
applying a force to the wall portion along a compression axis (CD) to compress the container and thereby provide an under-pressure in the container, the force causing less deformation of the wall portion along the compression axis than the resiliently deformable assembly's deformation along the compression axis.
